(19) Europäisches Patentamt
European Patent Office
Office européen des brevets

(11) Publication number: **0 016 349**
**A1**

(12) # EUROPEAN PATENT APPLICATION

(21) Application number: 80100886.3

(22) Date of filing: 22.02.80

(51) Int. Cl.³: C 07 D 211/70
A 61 K 31/445

(30) Priority: 02.03.79 US 16772

(43) Date of publication of application:
01.10.80 Bulletin 80/20

(84) Designated Contracting States:
AT BE CH DE FR GB IT LU NL SE

(71) Applicant: MERCK & CO. INC.
126, East Lincoln Avenue P.O. Box 2000
Rahway, New Jersey 07065(US)

(72) Inventor: Remy, David C.
607 Jenkins Lane, M.R. 1
North Wales, PA, 19454(US)

(74) Representative: Blum, Rudolf Emil Ernst et al,
c/o E. BLUM & CO. Vorderberg 11
CH-8044 Zürich(CH)

(54) **3-Formylcyproheptadine and analogs, process for their preparation and pharmaceutical compositions containing them.**

(57) 3-Formyl derivatives of cyproheptadine of the structural formula

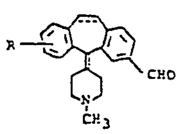

or a pharmaceutically acceptable salt thereof, wherein R is hydrogen, $C_{1-3}$ alkyl or fluoro, and the dotted line represents saturation or unsaturation, are appetite stimulants and antihistaminic agents.

EP 0 016 349 A1

Croydon Printing Company Ltd.

# 3-FORMYLCYPROHEPTADINE AND ANALOGS, PROCESS FOR THEIR PREPARATION AND PHARMACEUTICAL COMPOSITIONS CONTAINING THEM.

## BACKGROUND OF THE INVENTION

This invention is concerned with 3-formyl derivatives of cyproheptadine and related compounds which are appetite stimulants and antihistaminic agents.

Cyproheptadine itself and certain derivatives and analogs are known to be orexigenic and antihistaminic agents such as 3-carboxycyproheptadine in U.S. Patent 3,981,877, 3-carboxy-10,11-dihydrocyproheptadine in British Patent 1,486,847, 10-oxo (or hydroxy)-10,11-dihydrocyproheptadine in U.S. Patent 3,960,872.

Now with this invention there are provided further novel cyproheptadine derivatives and analogs carrying a 3-formyl group and novel processes for their preparation.

There is also provided a novel method of stimulating appetite and/or producing an antihistamine effect in patients in need of such treatment by the administration of one or more of the novel compounds or novel pharmaceutical compositions thereof which are also provided by this invention.

## DETAILED DESCRIPTION OF THE INVENTION

The novel compounds of this invention have structural formula:

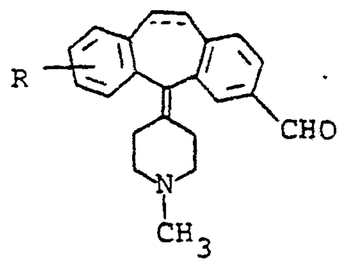

or a pharmaceutically acceptable salt thereof, wherein

R represents hydrogen, $C_{1-3}$ alkyl such as methyl, ethyl, propyl, especially methyl, or fluoro; and

the dotted line represents saturation or unsaturation of the molecule at the 10,11-position.

It is preferred that R be hydrogen.

The novel compounds of this invention with a 10,11-double bond demonstrate atropisomerism and exist as dextro- and levorotatory enantiomers.

The pharmaceutically acceptable salts of the novel compounds of this invention are acid addition salts formed from a novel compound and an organic or inorganic acid recognized by the art as providing a pharmaceutically acceptable acid addition salt, such as hydrochloride, hydrobromide, dihydrogen phosphate, sulfate, pamoate, citrate, napsylate, pyruvate, isethionate, maleate, fumarate, or the like.

The salts are prepared by dissolving approximately equimolecular amounts of the free base compound and the desired acid in a solvent followed by crystallization of the salt product.

The novel process of this invention comprises the oxidation of the corresponding 3-hydroxymethyl compound to the 3-formyl analog. The preferred oxidizing agent for this purpose is sulfur trioxide-trimethylamine complex in dimethylsulfoxide. The reaction is conducted at about 0° to 50°C but preferably at about ambient temperature, in the presence of a strong base such as a tri($C_{1-3}$ alkyl)amine, such as triethylamine, pyridine, or N-methylpiperidine or the like, over a period of about 6 to 24 hours.

In the method of treatment and pharmaceutical composition aspects of the present invention it is noted that the precise unit dosage form and dosage level depend upon the case history of the individual being treated and consequently are left to the discretion of the therapist. In general, however, the compounds of the present invention produce the desired effect of appetite stimulation when given at from about 0.01 to about 10.0 mg per kg body weight per day. The preferred form of delivery of the instant compounds for appetite stimulation of domestic animals is by solution in drinking water or performulated feedstuffs. For human and animal administration, any of the usual pharmaceutical oral forms may be employed such as tablets, elixirs and aqueous suspensions comprising from about 0.01 to about 10.0 mg of the compounds of this invention per kg body weight given daily. Thus, for example, tablets given 2-4 times per day comprising from about 0.5 to about 50 mg of the compounds of this invention are suitable for human treatment. Sterile solutions

(representatively given for human treatment) for injection comprising from about 0.1 to about 10.0 mg of the compounds of this invention given two to four times daily are also suitable means of delivery. When an antihistaminic effect is indicated, the above-recited dosage forms and levels are also appropriate.

EXAMPLE 1

1-Methyl-4-(10,11-dihydro-3-formyl-5H-dibenzo[a,d]-cyclohepten-5-ylidene)piperidine hydrochloride

Step A: Preparation of 1-methyl-4-(10,11-dihydro-3-ethoxycarbonyl-5H-dibenzo[a,d]cyclohepten-5-ylidene)piperidine

A solution of 5.0 g 1-methyl-4-(3-carboxy-10,11-dihydro-5H-dibenzo[a,d]cyclohepten-5-ylidene) piperidine hydrochloride in 200 ml of absolute ethanol was treated with 5 ml of boron trifluoride etherate. After stirring and refluxing for 18 hours the ethanol was evaporated *in vacuo* and the residue was partitioned between saturated aqueous sodium carbonate solution and ether. The ether phase was separated, washed with 3 x 100 ml of water, dried over anhydrous magnesium sulfate, filtered and evaporated to dryness. The residue, 5.4 g of 1-methyl-4-(10,11-dihydro-3-ethoxycarbonyl-5H-dibenzo[a,d]cyclohepten-5-ylidene)piperidine, after recrystallization from acetonitrile had m.p. 85-87°C.

Step B: Preparation of 1-methyl-4-(10,11-dihydro-3-hydroxymethyl-5H-dibenzo[a,d]cyclohepten-5-ylidene)piperidine

A solution of 5.3 g of the ethyl ester from Step A in 200 ml of ether was added dropwise over about 30 minutes to a stirred mixture of 0.53 g of $LiAlH_4$ and 50 ml of ether. After the addition was complete the mixture was stirred 1 hour at

ambient temperature, warmed to reflux and allowed to cool spontaneously to ambient temperature. With vigorous stirring a saturated aqueous solution of ammonium chloride was added alowly until a granular precipitate and a clear ether phase formed. Anhydrous magnesium sulfate was added. After stirring a short time the mixture was filtered and the filtrate was concentrated to dryness to give 2.28 g of 1-methyl-4-(10,11-dihydro-3-hydroxymethyl-5H-dibenzo[a,d]-cyclohepten-5-ylidene)piperidine, m.p. 178-181°C after recrystallization from acetonitrile.

<u>Step C</u>: <u>Preparation of 1-methyl-4-(10,11-dihydro-3-formyl-5H-dibenzo[a,d]cyclohepten-5-ylidene)-piperidine hydrochloride</u>

A stirred mixture of 1.0 g of 1-methyl-4-(10,11-dihydro-3-hydroxymethyl-5H-dibenzo[a,d]-cyclohepten-5-ylidene)piperidine and 6 ml of dimethylsulfoxide is treated with 4 ml of triethylamine, 6 ml of dimethylsulfoxide and 1.53 g of sulfur trioxide-trimethylamine complex. The mixture is stirred bigorously for about 12 hours. Water (500 ml) is added and the mixture is extracted with 2 x 100 ml of ether. The combined ether extracts are washed with 10 x 100 ml of water, dried over anhydrous magnesium sulfate, filtered and concentrated to dryness <u>in vacuo</u>. The oily residue was dissolved in 5 ml of 4<u>N</u> ethanolic HCl and the solution was concentrated to dryness. The residue was triturated with acetone and the crystalline product was collected, and dried to give 1-methyl-4-(10,11-dihydro-3-formyl-5H-dibenzo[a,d]-cyclohepten-5-ylidene)piperidine hydrochloride, m.p. 272-276°C. (dec.).

Employing the procedure of Example 1 but substituting for the 1-methyl-4-(10,11-dihydro-3-carboxy-5H-dibenzo[a,d]cyclohepten-5-ylidene)-piperidine hydrochloride used therein an equimolecular amount of 1-methyl-4-(3-carboxy-5H-dibenzo[a,d]-cyclohepten-5-ylidene)piperidine, there is produced 1-methyl-4-(3-formyl-5H-dibenzo[a,d]cyclohepten-5-ylidene)piperidine hydrochloride.

Similarly prepared are:

1-methyl-4-(10,11-dihydro-3-formyl-7-methyl-5H-dibenzo[a,d]cyclohepten-5-ylidene)piperidine;

1-methyl-4-(3-formyl-7-methyl-5H-dibenzo[a,d]-cyclohepten-5-ylidene)piperidine;

1-methyl-4-(10,11-dihydro-7-fluoro-3-formyl-5H-dibenzo[a,d]cyclohepten-5-ylidene)piperidine; and

1-methyl-4-(7-fluoro-3-formyl-5H-dibenzo[a,d]-cyclohepten-5-ylidene)piperidine.

## EXAMPLE 2

### Pharmaceutical Compositions

A typical tablet containing 1 mg 1-methyl-4-(10,11-dihydro-3-formyl-5H-dibenzo[a,d]cyclohepten-5-ylidene)piperidine hydrochloride per tablet is prepared by mixing together with the active ingredient calcium phosphate, lactose and starch in the amounts shown in the tables below. After these ingredients are thoroughly mixed, the appropriate amount of magnesium stearate is added and the dry mixture blended for an additional three minutes. This mixture is then compressed into tablets weighing approximately 124 mg each.

TABLE FORMULA

| INGREDIENT | MG. PER TABLET |
|---|---|
| 1-Methyl-4-(10,11-dihydro-3-formyl-5H-dibenzo[a,d]-cyclohepten-5-ylidene)-piperidine hydrochloride | 1 mg |
| Calcium phosphate | 52 mg |
| Lactose | 60 mg |
| Starch | 10 mg |
| Magnesium stearate | 1 mg |

WHAT IS CLAIMED IS:

1. A compound of structural formula:

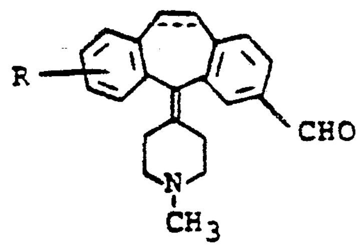

or a pharmaceutically acceptable salt thereof, wherein R is hydrogen, $C_{1-3}$ alkyl or fluoro, and the dotted line represents saturation or unsaturation.

2. The compound of Claim 1, wherein the compound is 1-methyl-4-(10,11-dihydro-3-formyl-5H-dibenzo[a,d]cyclohepten-5-ylidene)piperidine or 1-methyl-4-(3-formyl-5H-dibenzo[a,d]cyclohepten-5-ylidene)piperidine or a pharmaceutically acceptable salt thereof.

3. A pharmaceutical appetite stimulating or antihistaminic composition comprising a pharmaceutical carrier and an appetite stimulating or antihistaminic amount of a compound of structural formula:

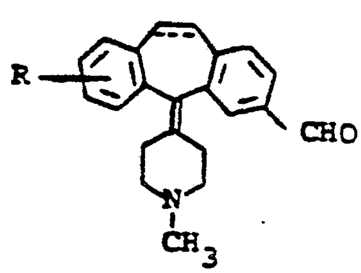

or a pharmaceutically acceptable salt thereof, wherein R is hydrogen, $C_{1-3}$ alkyl or fluoro, and the dotted line represents saturation or unsaturation.

4. The pharmaceutical appetite stimulating or antihistaminic composition of Claim 3, wherein the compound is 1-methyl-4-(10,11-dihydro-3-formyl-5H-dibenzo[a,d]cyclohepten-5-ylidene)piperidine or 1-methyl-4-(3-formyl-5H-dibenzo[a,d]cyclohepten-5-ylidene)piperidine or a pharmaceutically acceptable salt thereof.

5. A process for the preparation of a compound of structural formula:

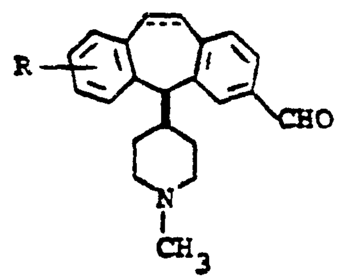

wherein R is hydrogen, $C_{1-3}$ alkyl or fluoro and the dotted line represents saturation or unsaturation comprising the oxidation of a compound of formula:

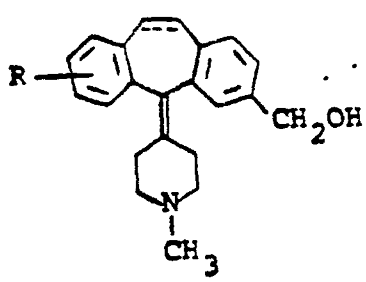

wherein $R^1$ is $C_{1-3}$ alkyl and R is as previously defined.

6. The process of Claim 5, for the preparation of 1-methyl-4-(10,11-dihydro-3-formyl-5H-dibenzo-[a,d]cyclohepten-5-ylidene)piperidine or 1-methyl-4-(3-formyl-5H-dibenzo[a,d]cyclohepten-5-ylidene)-piperidine or a pharmaceutically acceptable salt thereof.

0016349

European Patent Office

# EUROPEAN SEARCH REPORT

Application number

EP 80 10 0886

## DOCUMENTS CONSIDERED TO BE RELEVANT

| Category | Citation of document with indication, where appropriate, of relevant passages | Relevant to claim |
|---|---|---|
| A | DE - A - 3 851 059 (MERCK & CO.)<br>-- | |
| D,A | US - A - 3 960 872 (MERCK & CO.)<br>-- | |
| D,A | US - A - 3 981 877 (MERCK & CO.)<br>-- | |
| D,A | GB - A - 1 486 847 (MERCK & CO.)<br>---- | |

CLASSIFICATION OF THE APPLICATION (Int. Cl.3)

C 07 D 211/70
A 61 K 31/445

TECHNICAL FIELDS SEARCHED (Int.Cl.3)

A 61 K 31/445
C 07 D 211/70

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 22-05-1980 | FROELICH |

EPO Form 1503.1 06.78